# EUROPÄISCHE PATENTSCHRIFT

(11) **EP 3 235 422 B1**
(45) Veröffentlichungstag und Bekanntmachung des Hinweises auf die Patenterteilung: **03.06.2020**
(21) Anmeldenummer: 17175172.0
(22) Anmeldetag: 04.02.2010
(51) Int. Cl.: A61B 3/113

(54) **VERFAHREN UND VORRICHTUNG ZUR BESTIMMUNG DER AUGENDREHPUNKTLAGE**
DEVICE AND METHOD FOR DETERMINING THE EYE'S CENTRE OF ROTATION
PROCEDE ET DISPOSITIF DESTINES A LA DETERMINATION DE LA POSITION DU POINT ROTATIF OQULAIRE

(30) Priorität: 26.02.2009 DE 102009010467
(43) Veröffentlichungstag der Anmeldung: 25.10.2017
(62) Teilanmeldung aus: 12002380.9
(73) Patentinhaber: Carl Zeiss Vision GmbH, 73430 Aalen (DE)
(72) Erfinder: Kratzer, Timo, 73434 Aalen (DE); Cabeza-Guillén, Jesús-Miguel, 73434 Aalen (DE); Kelch, Gerhard, 73434 Aalen (DE)
(74) Vertreter: Glawe, Delfs, Moll

(56) Entgegenhaltungen:
- WO-A1-02/09025
- IMAI T ET AL: "Rotation vector analysis of eye movement in three dimensions with an infrared CCD camera", ACTA OTO-LARYNGOLO, SCANDINAVIAN UNIVERSITY PRESS, OSLO, NO, Bd. 119, Nr. 1, 1. Januar 1999 (1999-01-01), Seiten 24-28, XP008120861, ISSN: 0001-6489, DOI: 10.1080/00016489950181882

## Beschreibung

Die Erfindung bezieht sich auf Verfahren zur Bestimmung der Augendrehpunktlage nach dem Oberbegriff des Patentanspruchs 1.

Die Erfindung betrifft ferner eine Vorrichtung und ein Computerprogramm zum Durchführen des vorstehend genannten Verfahrens.

Es ist bekannt, zum Optimieren von Brillengläsern, insbesondere von individuellen Gleitsichtgläsern, verschiedene Parameter des Systems Auge/Brille zu berücksichtigen. Diese Parameter sind beispielsweise der Pupillenabstand PD, der Hornhaut-Scheitelabstand HSA, der Vorneigungswinkel α des Brillenglases, die Fassungsscheibenwinkel α_{R}, α_{L} für das rechte und linke Brillenglas sowie die Lage des optischen oder mechanischen Augendrehpunkts Z', M. Unter individuellen Gleitsichtgläsern versteht man Gleitsichtgläser, bei denen mindestens ein individueller Gebrauchsparameter bei der Berechnung des Brillenglases berücksichtigt wird und die mittels Freiformtechnologie gefertigt werden, wie dies z.B. in der Deutschen Optikerzeitung DOZ 4 + 5/2000 von W. Grimm und G. Kelch in dem Aufsatz "Gradal® Individual: Konzeption, Fertigung und Anpassung", in der EP 857 993 B1 und/oder der EP 562 336 B1 beschrieben ist.

Bei nicht-individuellen Brillengläsern werden diese Parameter aus statistischen Mittelwerten eines repräsentativen Bevölkerungsquerschnitts ermittelt. Bei individuellen Brillengläsern hingegen werden die Parameter individuell an dem jeweiligen Brillenträger gemessen, beispielsweise mit so genannten Videozentriersystemen, wie sie von der Anmelderin unter den Typenbezeichnungen "i.Terminal" und "Relaxed Vision Terminal" hergestellt und vertrieben werden. Diese Videozentriersysteme sind jedoch bisher nicht in der Lage, eine Optimierung des Brillenglases mit Hilfe der Augendrehpunktlage in einer ausreichend exakten Weise zu ermöglichen.

Wenn man die Augendrehpunktlage berücksichtigen möchte, kann man diese in bekannter Weise aus der Augenlänge über die mittlere Sphäre des verordneten Brillenglases ableiten. Dabei werden allerdings viele Annahmen gemacht, die mit den tatsächlichen Gegebenheiten nicht ausreichend übereinstimmen.

Der Zusammenhang zwischen Augenlänge und der Sphäre des verordneten Brillenglases wird oft vereinfacht als linear angenommen. Dies ist aber in der Realität nicht der Fall, weil sowohl die Krümmung der Hornhaut als auch die Augenlinse sowie die Augenlänge teilweise sehr unabhängig voneinander wachsen bzw. sich unterschiedlich entwickeln.

Im Allgemeinen ist es ausreichend, den Augendrehpunkt als punktförmiges Drehzentrum innerhalb des Auges zu betrachten. Die Erfindung umfasst jedoch ganz allgemein auch ein ausgedehntes, im Allgemeinen näherungsweise kugelförmiges Augendrehpunktsgebiet. Zur Beschreibung der Erfindung und des Standes der Technik wird nachfolgend der Einfachheit halber von einem punktförmigen Augendrehpunkt ausgegangen, soweit nichts Anderes erwähnt ist.

Üblicherweise geht man bei der Verordnung einer Brille so vor, dass die Brillengläser auf der Grundlage einer subjektiven Refraktion sowie einer Videozentriermessung verordnet und zentriert werden.

Dabei ist von Nachteil, dass es keine Referenzierung zwischen der Refraktionsbestimmung und der Zentrierung gibt, weil beide Vorgänge mit unterschiedlichen Apparaturen durchgeführt werden. Wenn die Refraktion beispielsweise mit einem Phoropter ermittelt wird, kann es vorkommen, dass der Phoropter um einen ersten Winkel relativ zu der der Linie verkippt ist, die die beiden Pupillenmittelpunkte des Auges verbindet. Ferner kann es vorkommen, dass die gewählte Brillenfassung relativ zu dieser Linie um einen zweiten Winkel verdreht ist. Im ungünstigsten Falle können sich die beiden Winkel addieren, was zu einer Diskrepanz von mehreren Winkelgraden in den Achsen der Zylinder zwischen der Verordnung und der ausgeführten Korrektur der fertigen Brille führen kann.

Aus der WO 2006/106248 A1 sind ein Verfahren und eine Vorrichtung zum Bestimmen der Augendrehpunktlage bekannt. Bei diesem bekannten Verfahren blickt ein Proband in ein fernrohrartiges Gerät. Die Ausrichtung des Gerätes im Raum wird durch einen am Gerät befindlichen, dreidimensional wirksamen Sensor bestimmt. Der Proband trägt an seinem Kopf einen weiteren derartigen Sensor, der die Lage und Ausrichtung seines Kopfes bestimmt. In dem Gerät befindet sich auf der vom Probanden abgewandten Seite eine Lichtquelle, die einen Lichtstrahl längs einer optischen Achse ausstrahlt. Zwischen der Lichtquelle und dem Auge des Probanden befinden sich zwei Gitter mit zentralen Marken. Der Proband bewegt das Gerät nun so lange, bis sich der Lichtstrahl und die beiden Marken decken. Aus den dabei ermittelten Positionsdaten für das Gerät und den Kopf wird die Lage der Blickachse im Raum errechnet. Der Vorgang wird dann mehrfach unter unterschiedlichen Blickrichtungen wiederholt, so dass mehrere Blickachsen bestimmt werden. Deren Drehpunkt wird dann als Augendrehpunktlage berechnet.

Diese bekannte Vorgehensweise hat den Nachteil, dass ein erheblicher Aufwand an separaten Geräten betrieben werden muss. Ferner ist die Messgenauigkeit vom subjektiven Verhalten des Probanden abhängig.

Aus der EP 0 825 826 A1 sind ein Verfahren und eine Vorrichtung zum parallelen Erfassen von Sehinformationen bekannt. Zum Bestimmen der Augendrehpunktlage wird bei dieser bekannten Vorgehensweise bei fester Ausrichtung und Lage des Kopfes des Probanden mindestens für zwei bekannte, nacheinander von den Augen fixierte Fixierpunkte eine Fixierlinien-Bestimmung durchgeführt. Die Fixierpunkte sind zwei fest mit der Halterung von Kameras und Sensoren zur Bestimmung der Pupillenlage verbindbare Markierpunkte. Der Augendrehpunkt liegt dann im Schnittpunkt der durch die Fixierpunkte gelegten Fixierlinien.

Der EP 1 154 302 A1 entnimmt man ebenfalls ein Verfahren zur Bestimmung des Augendrehpunkts aus dem Schnittpunkt der Fixierlinien für unterschiedliche Blickrichtungen.

Die WO 02/09025 A1 beschreibt ein Verfahren zur Bestimmung des Augendrehpunkts unter Berücksichtigung der Bestimmung der 2D Position für das Iris/Pupillen-Zentrum. WO 02/09025 A1 berücksichtigt nicht die Form der Pupille und/oder des Limbus und/oder der Iris und/oder andere biometrische Bestandteile des Auges bei mindestens zwei Blickrichtungen zur Bestimmung der Augendrehpunktlage.

Der Erfindung liegt die Aufgabe zugrunde, andere Verfahren und Vorrichtungen zur Bestimmung der Augendrehpunktlage zur Verfügung zu stellen. Die Augenlänge ist die geometrische Länge des Auges zwischen Hornhautscheitel und der Fovea. Unter Augendrehpunktlage versteht man ganz allgemein den Ort des optischen Augendrehpunkts. Als optischer Augendrehpunkt (Kurzzeichen Z') wird z.B. nach der DIN 5340-43 der Fußpunkt des Lotes vom mechanischen Augendrehpunkt auf die ins Augeninnere verlängerte Fixierlinie beim Blick geradeaus auf einen unendlich fernen Punkt bei ungezwungener Kopf- und Körperhaltung angenommen. Der mechanische Augendrehpunkt (Kurzzeichen M) ist z.B. nach der DIN 5340 ⁻ 42 derjenige Punkt im Auge, der sich bei Blickbewegungen am wenigsten verlagert.

Die der Erfindung zugrunde liegende Aufgabe wird gelöst durch ein Verfahren, wie in Anspruch 1 definiert und durch eine Vorrichtung, wie in Anspruch 14 definiert. Die Aufnahmeeinrichtung kann z.B. eine Zentriereinheit oder eine Digitalkamera sein. Die Ermittlungseinrichtung und die Augendrehpunkt-Bestimmungseinrichtung können z.B. gemeinsam in Form eines handelsüblichen Personal Computers realisiert sein.

Die der Erfindung zugrunde liegende Aufgabe wird auf diese Weise vollkommen gelöst.

Das erfmdungsgemäße Verfahren zeichnet sich dadurch aus, dass die Augendrehpunktlage noch exakter als herkömmlich bestimmt werden kann. Dadurch ist eine noch bessere Optimierung insbesondere von individuellen Gleitsichtgläsern möglich, wenn man die Augendrehpunktlage bei der Berechnung und Herstellung der Oberflächentopographie der individuellen Gleitsichtgläser berücksichtigt.

Durch das erfindungsgemäße Verfahren wird es möglich, die vorstehend genannte noch bessere Optimierung mittels einer nur geringfügigen Modifizierung bekannter Systeme zu erreichen.

Zusätzlich zur Form des charakteristischen Bestandteils des Auges kann auch dessen Lage erfasst und bei der Ermittlung der charakteristischen Achse des Auges berücksichtigt werden.

Bei dem Verfahren kann der charakteristische Bestandteil des Auges z.B. die Pupille und/oder den Limbus und/oder die Iris umfassen. Diese charakteristischen Bestandteile des Auges sind mit bloßem Auge erkennbar und können daher in einfacher Weise sowohl von einem Benutzer als auch von einem automatischen Erfassungssystem eindeutig identifiziert werden. Eine Fehlinterpretation ist daher weitgehend ausgeschlossen.

Anstelle der vorstehend angegebenen charakteristischen Augenbestandteile Pupille, Limbus und/oder Iris können selbstverständlich auch andere für das Auge charakteristische (insbesondere biometrische) Bestandteile, wie z.B. Äderchen oder in der Farbe unterscheidbare Bereiche, verwendet werden. Derartige Strukturen können sogar im Einzelfall bevorzugt sein, z.B. wenn es auf die fehlende Invarianz der Struktur gegenüber Drehungen ankommt.

Die Lage und/oder die Form des charakteristischen Bestandteils des Auges kann z.B. durch ein kalibriertes photographisches System erfasst werden. Unter kalibriertem photographischem System versteht man ein photographisches System welches zur Erfassung von dreidimensionalen Parametern des Kopf-Auge-Systems verwendet werden kann. Die Verwendung eines derartigen Systems hat den Vorteil, dass man damit mit ausreichender Genauigkeit messen kann.

Als kalibriertes photographisches System kann z.B. ein kalibriertes Videozentriersystem verwendet werden. Ein Videozentriersystem ohne Kalibrierung ist i.A. nur eine Digitalkamera und somit wertlos bei der Messung von Gebrauchsparametern.

Die wenigstens eine charakteristische Achse, deren Lage für die zwei Blickrichtungen aus der jeweiligen Lage und/oder der jeweiligen Form des charakteristischen Bestandteils des Auges bei den beiden Blickrichtungen ermittelt wird, kann z.B. die Fixierlinie und/oder die Sehachse und/oder die optischen Achse umfassen. Alle drei charakteristischen können z.B. nach dem o.a. Verfahren durch einfache Rechenoperationen aus den zuvor aufgenommenen Daten bestimmt werden.

Bei diesem Verfahren kann die Bestimmung der Augendrehpunktlage eine Bestimmung des Schnittorts der charakteristischen Achsen des Auges umfassen.

Dieses Verfahren kann z.B. folgende Schritte umfassen:
a) Erfassen des charakteristischen Bestandteils des Auges und Ermitteln dessen geometrischen Mittelpunktes sowie der Normalen in der Ebene des charakteristischen Bestandteils in dem Mittelpunkt bei einer ersten Blickrichtung des Probanden;
b) Erfassen des charakteristischen Bestandteils des Auges und Ermitteln dessen geometrischen Mittelpunktes sowie der Normalen in der Ebene des charakteristischen Bestandteils in dem Mittelpunkt bei einer zweiten von der ersten Blickrichtung abweichenden Blickrichtung des Probanden; und
c) Bestimmen der Augendrehpunktlage aus den Richtungsvektoren der in den Schritten a) und b) ermittelten Normalen.

Anstelle des Schnittorts einer charakteristischen Achse bei unterschiedlichen Blickrichtungen kann man auch zwei charakteristische Achsen ermitteln und die Augendrehpunktlage als einen Schnittpunkt der beiden charakteristischen Achsen bestimmen.

Anstelle des Schnittorts zweier charakteristischer Achsen können auch mehr als zwei charakteristische Achsen ermittelt werden und die Augendrehpunktlage als Mittelpunkt eines von den charakteristischen Achsen tangential umschlossenen Kugelvolumens bestimmt werden.

Ein Verfahren zum Optimieren eines für ein Auge eines Probanden individuellen Brillenglases, bei dem die Augendrehpunktlage ermittelt und als Eingangsparameter verwendet wird, kann sich zur Bestimmung der Augendrehpunktlage in dem Auge des Probanden des vorstehend beschriebenen Verfahrens bedienen.

Ein Computerprogramm mit Programmcode kann zur Durchführung desvorstehend beschriebenen Verfahrens eingerichtet sein, wenn das Programm in einem Computer ausgeführt wird. Das Computerprogramm kann z.B. auf einem maschinenlesbaren Datenträger gespeichert sein.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Figur 1: eine erste schematische Seitenansicht eines nach oben verdrehten Auges zur Erläuterung von verschiedenen Blickrichtungen und Drehpunkten und der Lage des Augendrehpunktes;
- Figur 2: eine zweite schematische Seitenansicht eines geradeaus gerichteten Auges mit vorgesetztem Brillenglas zur Erläuterung weiterer Parameter des Auges;
- Figur 3: eine Frontansicht eines Auges zur Erläuterung bestimmter Augenbereiche;
- Figur 4: eine Vorrichtung zur Bestimmung der Augendrehpunktlage nach der Erfindung.

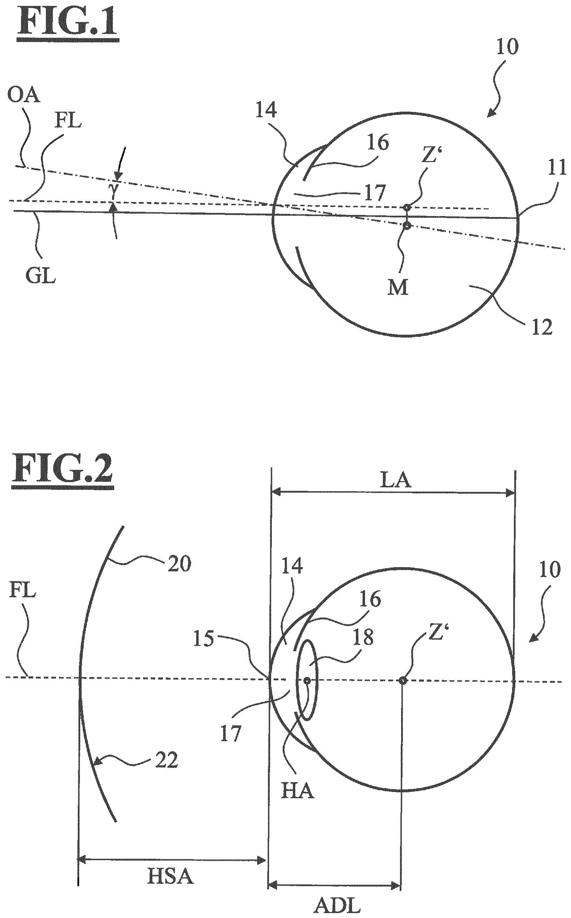

In den Figuren 1 und 2 bezeichnet 10 ein Auge. Das Auge 10 hat einen Glaskörper 12, eine Hornhaut 14, eine Iris 16, eine Pupille 17 sowie eine Linse 18.

Wenn das Auge 10 eine Drehbewegung ausführt, dann geschieht dies nicht exakt um einen Drehpunkt im Raum. Vielmehr gibt es nur einen näherungsweise kugelförmigen Bereich, in dem sich die momentanen Drehpunkte befinden. Derjenige Punkt, der bei Augenbewegungen die geringste Lageveränderung erfährt, wird als mechanischer Augendrehpunkt M bezeichnet (vgl. DIN 5340 - 42).

Mit GL ist die Sehachse (Gesichtslinie) bezeichnet. Sie ist nach DIN 5340 - 360 die Verbindungsgerade zwischen einem fixierten Objektpunkt und dem dazu konjugierten Bildpunkt in der Mitte der Netzhautgrube 11.

Mit FL ist die Fixierlinie (Visierlinie) bezeichnet. Sie ist nach DIN 5340 - 159 die Verbindungsgerade zwischen dem zentral abgebildeten Objektpunkt und der Mitte der Eintrittspupille 17.

OA bezeichnet die optische Achse.

Mit Z' ist der optische Augendrehpunkt bezeichnet. Er ist nach DIN 5340 - 43 der Fußpunkt des Lotes vom mechanischen Augendrehpunkt M auf die Fixierlinie FL.

Der Winkel zwischen der optischen Achse OA und der Fixierlinie FL ist in Figur 1 mit γ bezeichnet. Der Winkel γ ist hier nur in einer Ebene eingezeichnet, symbolisiert aber einen Raumwinkel oben/unten und /rechts/links.

In Figur 2 ist ein Brillenglas 20 vor dem Auge 10 angeordnet. Das Brillenglas hat auf der dem Auge 10 zugewandten Seite eine Rückfläche 22. Der Abstand der Rückfläche 22 von dem Hornhaut-Apex 15 gemessen in Blickrichtung senkrecht zur Fassungsebene wird als Hornhaut-Scheitelabstand HSA bezeichnet (vgl. DIN EN ISO 13666 - 5.27). Der Abstand des Hornhautscheitels 15 vom optischen Augendrehpunkt Z' gibt die Augendrehpunktlage ADL in Bezug auf den Hornhaut-Apex 15 an.

Die Augendrehpunktlage ADL ist ein wichtiger Parameter in der Berechnung des Brillenglases 20. Das Brillenglas 20 wird immer so optimiert, dass es für jede Blickrichtung des Auges 10 die optimalen Abbildungseigenschaften aufweist.

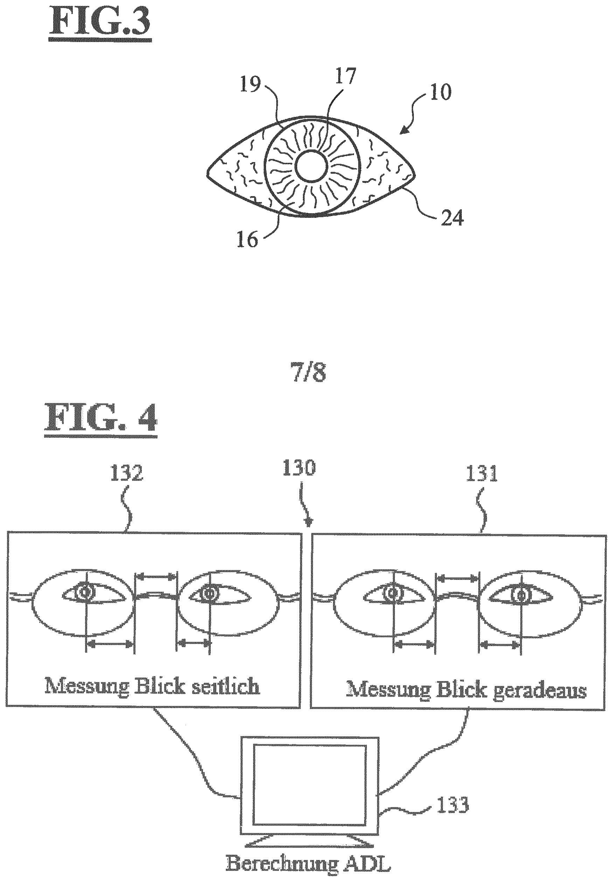

Figur 3 zeigt eine Frontansicht des Auges 10. Man erkennt in der Iris 17 eine charakteristische Struktur sowie neben der Iris 17 in der Lederhaut 24 eine Struktur von kleinen Blutgefäßen.

### Ausführungsbeispiel:

Gemäß einem Ausführungsbeispiel eines erfindungsgemäßen Verfahrens ist es möglich, Form und gegebenenfalls Lage eines charakteristischen Bestandteils des Auges bei mindestens zwei Blickrichtungen zu erfassen und aus diesen wiederum die Lage wenigstens einer charakteristischen Achse des Auges für diese zwei Blickrichtungen zu ermitteln und anhand dieser charakteristischen Achse(n) des Auges für die beiden Blickrichtungen die Augendrehpunktlage zu bestimmen.

Die derart bestimmte Augendrehpunktlage kann dann als Eingangsparameter in der Berechnung eines individuell optimierten Brillenglases verwendet werden.

Die erste in Kombination mit der zweiten Variante erfindungsgemäße Variante, nämlich die Bestimmung der Augendrehpunktlage aus der Lage eines charakteristischen Bestandteils des Auges, ergibt sich wie folgt:
Dem Probanden wird aufgetragen, ein bestimmtes Fixierziel anzusehen. Es erfolgt eine Aufnahme des Auges für diese Blickrichtung mit Hilfe eines kalibrierten photographischen Systems. Ein derartiges kalibriertes photographisches System kann ein Video-Zentriersystem sein, wie es von der Anmelderin unter den Bezeichnungen RVT und i.Terminal vertrieben wird. Zu diesem Zweck muss das Video-Zentriersystem lediglich so eingesetzt werden, dass es Bilder des Auges 10 in unterschiedlichen Blickrichtungen aufnehmen kann. Das Bezugszeichen 131 in Figur 4 kennzeichnet eine Aufnahme eines derartigen Videozentriersystems für die Blickrichtung geradeaus.

Dann wird dem Probanden aufgetragen, ein Fixierziel in einer anderen Blickrichtung anzusehen. Es erfolgt eine erneute Aufnahme des Auges für diese zweite Blickrichtung mit Hilfe des kalibrierten photographischen Systems. Das Bezugszeichen 132 in Figur 4 kennzeichnet eine Aufnahme des Videozentriersystems für die Blickrichtung seitlich. Aus diesen Aufnahmen wird dann die Lage des charakteristischen Augenbestandteils, wie z.B. der Pupille (insbesondere der Pupillenmitte), der Iris, des Limbus, eines Blutgefässes oder dergleichen z.B. mittels eines Computers 133 ermittelt. Aus dem Wissen über die unterschiedlichen Blickrichtungen γ, d.h. der Bekanntheit des jeweiligen Fixierpunkts, und der jeweiligen Lage des aus den Aufnahmen gewonnenen charakteristischen Augenbestandteils lassen sich jeweils charakteristische Augenachsen, wie z.B. die Fixierlinie FL, und/oder die Sehachse GL und/oder die optische Achse OA für die unterschiedlichen Blickrichtungen γ ermitteln. Diese dienen dann zur Ermittlung der Augendrehpunktlage ADL.

Konkret wird beispielsweise in einem ersten Schritt 2a) eine Aufnahme der Lage der Pupille 17 und der Pupillenmitte gemacht, wobei der Proband z.B. geradeaus blickt. Daraus wird die Normale zur Pupillenebene in der Pupillenmitte und damit eine erste Blickrichtung γ₁ ermittelt.

In einem zweiten Schritt 2b) wird eine Aufnahme der Lage der Pupille 17 und der Pupillenmitte gemacht, wobei der Proband nunmehr seitlich blickt. Daraus wird wiederum die Normale zur Pupillenebene in der Pupillenmitte und damit eine zweite Blickrichtung γ₂ ermittelt.

In einem dritten Schritt 2c) wird nun aus den beiden unterschiedlichen Blickrichtungen γ₁, γ₂ der Augendrehpunkt Z', d.h. die Augendrehpunktlage ADL, als Schnittpunkt der beiden Normalvektoren ermittelt.

Bei Aufnahmen von mehr als zwei Blickrichtungen γ kann auch das bereits erwähnte ausgedehnte, im Allgemeinen näherungsweise kugelförmige Gebiet bestimmt werden, in dem sich die momentanen Augendrehpunkte befinden.

Die zweite Variante, nämlich die Bestimmung der Augendrehpunktlage aus der Form eines charakteristischen Bestandteils des Auges, wird nachfolgend beschrieben:
Wie bei der vorangegangenen Variante erfolgt die Aufnahme des Auges für unterschiedliche Blickrichtungen γ mit Hilfe eines kalibrierten photographischen Systems. Aus den Aufnahmen wird dann die Form des charakteristischen Augenbestandteils, wie z.B. der Pupille, der Iris, der Blutgefäße oder dergleichen ermittelt. Aus diesen Formen lassen sich charakteristische Achsen ableiten. Anders als bei der vorstehend beschriebenen Variante ist es bei dieser Verfahrensvariante nicht erforderlich die unterschiedlichen Fixierziele bei den unterschiedlichen Blickrichtungen γ zu kennen. Die Achsen dienen dann wiederum zur Ermittlung der Augendrehpunktlage.

Die Augendrehpunktlage wird demgemäß aus einer mathematischen Transformation am Auge 10 bei unterschiedlicher Blickrichtung γ bestimmt und für die mathematische Transformation ein kalibriertes photographisches System verwendet.

### Bezugszeichenliste

- 10: Auge
- 11: Netzhautgrube
- 12: Glaskörper
- 13: Netzhautebene
- 14: Hornhaut
- 15: Hornhautscheitel
- 16: Iris
- 17: Pupille
- 18: Linse
- 19: Limbus
- 20: Brillenglas
- 22: Rückfläche
- 24: Lederhaut
- 130: Vorrichtung zur Bestimmung der Augendrehpunktlage
- 131: Videozentriergerät (Aufnahme 1)
- 132: Videozentriergerät (Aufnahme 2)
- 133: Computer

- ADL: Augendrehpunktlage
- HA: objektseitiger Hauptpunkt des Auges
- HSA: Hornhaut-Scheitelabstand
- FL: Fixierlinie
- GL: Sehachse
- LA: Augenlänge
- M: mechanischer Augendrehpunkt
- OA: optische Achse
- Z': optischer Augendrehpunkt
- x, y, z: Raumkoordinaten

- γ: Winkel, Blickrichtung

## Patentansprüche

1. Verfahren zur Bestimmung der Augendrehpunktlage (ADL) in Bezug auf den Hornhautscheitel in einem Auge (10) eines Probanden, **gekennzeichnet durch** die Schritte:
a) Erfassen der Form eines charakteristischen Bestandteils des Auges (10) durch eine Aufnahmeeinrichtung (131, 132), bei mindestens zwei Blickrichtungen (γ);
b) Ermitteln der Lage wenigstens einer charakteristischen Achse (OA, FL, GL) des Auges (10) für die zwei Blickrichtungen (γ) aus der jeweils erfassten Form des charakteristischen Bestandteils des Auges (10) bei den zwei Blickrichtungen (γ);
c) Bestimmen der Augendrehpunktlage (ADL) aus der Lage der charakteristischen Achse (OA, FL, GL) des Auges (10) für die zwei Blickrichtungen (γ);
wobei die Schritte b) und c) mittels eines Computers ausgeführt werden.

2. Verfahren nach Anspruch 1, **dadurch gekennzeichnet, dass** der Schritt a) ein Erfassen der Lage des charakteristischen Bestandteils des Auges (10) bei den mindestens zwei Blickrichtungen (γ) umfasst und dass der Schritt b) ein Ermitteln der Lage der wenigstens einen charakteristischen Achse (OA, FL, GL) des Auges (10) für die zwei Blickrichtungen (γ) aus der Lage des charakteristischen Bestandteils des Auges (10) umfasst.

3. Verfahren nach Anspruch 1 oder 2, **dadurch gekennzeichnet, dass** der charakteristische Bestandteil des Auges (10) die Pupille und/oder den Limbus und/oder die Iris umfasst.

4. Verfahren nach einem der vorangegangenen Ansprüche, **dadurch gekennzeichnet, dass** die Lage und die Form des charakteristischen Bestandteils des Auges (10) durch ein kalibriertes photographisches System erfasst werden.

5. Verfahren nach Anspruch 4, **dadurch gekennzeichnet, dass** als kalibriertes photographisches System ein Videozentriersystem verwendet wird.

6. Verfahren nach einem der vorangegangenen Ansprüche, **dadurch gekennzeichnet, dass** die wenigstens eine charakteristische Achse die Fixierlinie (FL) und/oder die Sehachse (SA) und/oder die optische Achse (OA) umfasst.

7. Verfahren nach einem der vorangegangenen Ansprüche, **dadurch gekennzeichnet, dass** die Bestimmung der Augendrehpunktlage (ADL) eine Bestimmung des Schnittorts (Z') der charakteristischen Achsen (OA; GL; FL) des Auges (10) umfasst.

8. Verfahren nach einem der vorangegangenen Ansprüche, **gekennzeichnet durch** die Schritte:
a) Erfassen des charakteristischen Bestandteils (17) des Auges (10) und Ermitteln dessen geometrischen Mittelpunktes sowie der Normalen in der Ebene des charakteristischen Bestandteils in dem Mittelpunkt bei einer ersten Blickrichtung des Probanden;
b) Erfassen des charakteristischen Bestandteils (17) des Auges (10) und Ermitteln dessen geometrischen Mittelpunktes sowie der Normalen in der Ebene des charakteristischen Bestandteils in dem Mittelpunkt bei einer zweiten von der ersten Blickrichtung abweichenden Blickrichtung des Probanden;
c) Bestimmen der Augendrehpunktlage (ADL) aus den Richtungsvektoren der in den Schritten a) und b) ermittelten Normalen.

9. Verfahren nach einem der Ansprüche 7 oder 8, **dadurch gekennzeichnet, dass** zwei charakteristische Achsen ermittelt und die Augendrehpunktlage (ADL) als ein Schnittpunkt der beiden charakteristischen Achsen bestimmt wird.

10. Verfahren nach einem der Ansprüche 7 oder 8, **dadurch gekennzeichnet, dass** mehr als zwei charakteristische Achsen ermittelt und die Augendrehpunktlage (ADL) als Mittelpunkt eines von den charakteristischen Achsen tangential umschlossenen Kugelvolumens bestimmt wird.

11. Verfahren zum Optimieren eines für ein Auge (10) eines Probanden individuellen Brillenglases (20), bei dem die Augendrehpunktlage (ADL) ermittelt und als Eingangsparameter verwendet wird, **dadurch gekennzeichnet, dass** zur Bestimmung der Augendrehpunktlage (ADL) in dem Auge (10) des Probanden ein Verfahren nach einem der vorangegangenen Ansprüche verwendet wird.

12. Computerprogramm mit Programmcode eingerichtet zur Durchführung des Verfahrens nach einem der vorangegangenen Ansprüche, wenn das Programm in einem Computer ausgeführt wird, welcher eine Vorrichtung gemäss Anspruch 14 steuert.

13. Computerprogramm nach Anspruch 12, gespeichert auf einem maschinenlesbaren Datenträger.

14. Vorrichtung (130) zur Bestimmung der Augendrehpunktlage (ADL) in Bezug auf den Hornhautscheitel in einem Auge (10) eines Probanden, **gekennzeichnet durch**:
a) eine Aufnahmeeinrichtung (131, 132) zum Erfassen der Form eines charakteristischen Bestandteils des Auges (10) bei mindestens zwei Blickrichtungen (γ);
b) eine Ermittlungseinrichtung (133) zum Ermitteln der Lage wenigstens einer charakteristischen Achse (OA, FL, GL) des Auges (10) für die zwei Blickrichtungen (γ) aus der jeweils erfassten Form des charakteristischen Bestandteils des Auges (10) bei den zwei Blickrichtungen;
c) eine Bestimmungseinrichtung (133) zum Bestimmen der Augendrehpunktlage (ADL) aus der Lage der charakteristischen Achse (OA, FL, GL) des Auges (10) für die zwei Blickrichtungen (γ).

## Claims

1. Method for determining the eye's centre of rotation (ADL) in relation to the corneal apex in an eye (10) of a test subject, **characterized by** the steps of:
a) detecting the shape of a characteristic part of the eye (10) by means of a recording device (131, 132) in at least two viewing directions (γ);
b) ascertaining the position of at least one characteristic axis (OA, FL, GL) of the eye (10) for the two viewing directions (γ) from the shape of the characteristic part of the eye (10) respectively detected in the two viewing directions (γ);
c) determining the eye's centre of rotation (ADL) from the position of the characteristic axis (OA, FL, GL) of the eye (10) for the two viewing directions (γ);
wherein steps b) and c) are executed by means of a computer.

2. Method according to Claim 1, **characterized in that** step a) comprises detecting the position of the characteristic part of the eye (10) in the at least two viewing directions (γ) and **in that** step b) comprises ascertaining the position of the at least one characteristic axis (OA, FL, GL) of the eye (10) for the two viewing directions (γ) from the position of the characteristic part of the eye (10).

3. Method according to Claim 1 or 2, **characterized in that** the characteristic part of the eye (10) comprises the pupil and/or the limbus and/or the iris.

4. Method according to any of the preceding claims, **characterized in that** the position and the shape of the characteristic part of the eye (10) are detected by means of a calibrated photographic system.

5. Method according to Claim 4, **characterized in that** a video centration system is used as a calibrated photographic system.

6. Method according to any of the preceding claims, **characterized in that** the at least one characteristic axis comprises the fixation line (FL) and/or the visual axis (SA) and/or the optical axis (OA).

7. Method according to any of the preceding claims, **characterized in that** determining the eye's centre of rotation (ADL) comprises determining the intersection location (Z') of the characteristic axes (OA; GL; FL) of the eye (10).

8. Method according to any of the preceding claims, **characterized by** the steps:
a) detecting the characteristic part (17) of the eye (10) and ascertaining the geometric centre thereof and the normal in the plane of the characteristic part at the centre in a first viewing direction of the test subject;
b) detecting the characteristic part (17) of the eye (10) and ascertaining the geometric centre thereof and the normal in the plane of the characteristic part at the centre in a second viewing direction, deviating from the first viewing direction, of the test subject;
c) determining the eye's centre of rotation (ADL) from the direction vectors of the normals ascertained in steps a) and b).

9. Method according to either of Claims 7 and 8, **characterized in that** two characteristic axes are ascertained, and the eye's centre of rotation (ADL) is determined as an intersection point of the two characteristic axes.

10. Method according to either of Claims 7 and 8, **characterized in that** more than two characteristic axes are ascertained, and the eye's centre of rotation (ADL) is determined as the centre of a spherical volume tangentially enclosed by the characteristic axes.

11. Method for optimizing a spectacle lens (20) customized for an eye (10) of a test subject, in which the eye's centre of rotation (ADL) is ascertained and used as an input parameter, **characterized in that** a method according to any of the preceding claims is used in determining the eye's centre of rotation (ADL) in the eye (10) of the test subject.

12. Computer program with program code set up to carry out the method according to any of the preceding claims when the program is executed in a computer, which controls an apparatus according to Claim 14.

13. Computer program according to Claim 12, stored on a machine-readable data medium.

14. Apparatus (130) for determining the eye's centre of rotation (ADL) in relation to the corneal apex in an eye (10) of a test subject, **characterized by**:
a) a recording device (131, 132) for detecting the shape of a characteristic part of the eye (10) in at least two viewing directions (γ);
b) an ascertaining device (133) for ascertaining the position of at least one characteristic axis (OA, FL, GL) of the eye (10) for the two viewing directions (γ) from the shape of the characteristic part of the eye (10) respectively detected in the two viewing directions (γ);
c) a determination device (133) for determining the eye's centre of rotation (ADL) from the position of the characteristic axis (OA, FL, GL) of the eye (10) for the two viewing directions (γ).

## Revendications

1. Procédé de détermination de la position du centre de rotation de l'œil (ADL) en référence au sommet de la cornée dans un œil (10) d'un sujet, **caractérisé par** les étapes suivantes :
a) détection de la forme d'un élément constitutif caractéristique de l'œil (10) par un dispositif d'enregistrement (131, 132), pour au moins deux directions de visée (γ);
b) identification de la position d'au moins un axe caractéristique (OA, FL, GL) de l'œil (10) pour les deux directions de visée (γ) à partir de la forme respectivement détectée de l'élément constitutif caractéristique de l'œil (10) pour les deux directions de visée (γ);
c) détermination de la position du centre de rotation de l'œil (ADL) à partir de la position de l'axe caractéristique (OA, FL, GL) de l'œil (10) pour les deux directions de visée (γ) ;
les étapes b) et c) étant exécutées au moyen d'un ordinateur.

2. Procédé selon la revendication 1, **caractérisé en ce que** l'étape a) comprend une détection de la position de l'élément constitutif caractéristique de l'œil (10) pour les au moins deux directions de visée (γ) et **en ce que** l'étape b) comprend une identification de la position de l'au moins un axe caractéristique (OA, FL, GL) de l'œil (10) pour les deux directions de visée (γ) à partir de la position de l'élément constitutif caractéristique de l'œil (10).

3. Procédé selon la revendication 1 ou 2, **caractérisé en ce que** l'élément constitutif caractéristique de l'œil (10) comprend la pupille et/ou le limbe et/ou l'iris.

4. Procédé selon l'une des revendications précédentes, **caractérisé en ce que** la position et la forme de l'élément constitutif caractéristique de l'œil (10) sont détectées par un système photographique étalonné.

5. Procédé selon la revendication 4, **caractérisé en ce que** le système photographique étalonné utilisé est un système de centrage vidéo.

6. Procédé selon l'une des revendications précédentes, **caractérisé en ce que** l'au moins un axe caractéristique comprend la ligne de fixation (FL) et/ou l'axe visuel (SA) et/ou l'axe optique (OA).

7. Procédé selon l'une des revendications précédentes, **caractérisé en ce que** la détermination de la position du centre de rotation de l'œil (ADL) comprend une détermination du lieu d'intersection (Z') des axes caractéristiques (OA, FL, GL) de l'œil (10).

8. Procédé selon l'une des revendications précédentes, **caractérisé par** les étapes suivantes :
a) détection de l'élément constitutif caractéristique (17) de l'œil (10) et identification de son centre géométrique ainsi que de la normale dans le plan de l'élément constitutif caractéristique au centre pour une première direction de visée du sujet ;
b) détection de l'élément constitutif caractéristique (17) de l'œil (10) et identification de son centre géométrique ainsi que de la normale dans le plan de l'élément constitutif caractéristique au centre pour une deuxième direction de visée du sujet différente de la première direction de visée ;
c) détermination de la position du centre de rotation de l'œil (ADL) à partir des vecteurs de direction des normales identifiées aux étapes a) et b).

9. Procédé selon l'une des revendications 7 ou 8, **caractérisé en ce que** deux axes caractéristiques sont identifiés et la position du centre de rotation de l'œil (ADL) est déterminée comme un point d'intersection des deux axes caractéristiques.

10. Procédé selon l'une des revendications 7 ou 8, **caractérisé en ce que** plus de deux axes caractéristiques sont identifiés et la position du centre de rotation de l'œil (ADL) est déterminée comme le centre d'un volume sphérique cerné tangentiellement par les axes caractéristiques.

11. Procédé d'optimisation d'un verre de lunettes (20) personnalisé pour un œil (10) d'un sujet, avec lequel la position du centre de rotation de l'œil (ADL) est identifiée et utilisée comme paramètre d'entrée, **caractérisé en ce qu'**un procédé selon l'une des revendications précédentes est utilisé pour la détermination de la position du centre de rotation de l'œil (ADL) dans l'œil (10) du sujet.

12. Programme informatique comprenant un code de programme conçu pour mettre en œuvre un procédé selon l'une des revendications précédentes lorsque le programme est exécuté dans un ordinateur, lequel commande un arrangement selon la revendication 14.

13. Programme informatique selon la revendication 12, enregistré sur un support de données lisible par machine.

14. Arrangement (130) de détermination de la position du centre de rotation de l'œil (ADL) en référence au sommet de la cornée dans un œil (10) d'un sujet, **caractérisé par** :
a) un dispositif d'enregistrement (131, 132) destiné à détecter la forme d'un élément constitutif caractéristique de l'œil (10) pour au moins deux directions de visée (γ) ;
b) un dispositif d'identification (133) destiné à identifier la position d'au moins un axe caractéristique (OA, FL, GL) de l'œil (10) pour les deux directions de visée (γ) à partir de la forme respectivement détectée de l'élément constitutif caractéristique de l'œil (10) pour les deux directions de visée ;
c) un dispositif de détermination (133) destiné à déterminer la position du centre de rotation de l'œil (ADL) à partir de la position de l'axe caractéristique (OA, FL, GL) de l'œil (10) pour les deux directions de visée (γ).
